# EUROPEAN PATENT APPLICATION

(11) **EP 1 797 844 A1**
(43) Date of publication of application: **20.06.2007**
(21) Application number: 06126028.7
(22) Date of filing: 13.12.2006
(51) Int. Cl.: A61F 2/06, A61L 31/16

(54) **An endovascular device with membrane having permanently attached agents**

(30) Priority: 13.12.2005 SG 200508026
(71) Applicant: Merlin MD PTE Ltd, Singapore 757716 (SG)
(72) Inventor: Rudakov, Leon, Belmont, CA 94002 (US); Hong, Tsui Ying, Rachel, 670603 Singapore (MY); Sung, Peir Fen, 210048 Singapore (MY)
(74) Representative: Vogel, Andreas

(57) **Abstract**

An endovascular device (10) for treatment of a bodily vessel (5) of a patient at a surgical site, the endovascular device (10) comprising: a mechanically expandable device (11) expandable from a first position to a second position, said mechanically expandable device (11) is expanded radially outwardly to the second position such that the circumferential surface of said mechanically expandable device (11) engages with the inner surface of the vessel (5) so as to maintain a fluid pathway through said vessel (5); and a membrane (15) secured to a portion of the circumferential surface of said mechanically expandable device (11); wherein at least one capture agent (20, 21) is permanently attached to the lumenal surface of the membrane (15) to capture a predetermined target component from blood passing through the fluid pathway; and at least one signal agent (22) is permanently attached to the lumenal surface of the membrane (15) to signal the captured target component to up regulate or down regulate a cell function of the captured target component to enhance endothelialization and healing.

## Description

### Technical Field

The invention concerns an endovascular device for treatment of a bodily vessel of a patient at a surgical site.

### Background of the Invention

The placement of an endovascular stenting device in a bodily vessel induces thrombosis, inflammatory reactions and subsequently smooth muscle cell proliferation. This results in occlusion of the vessel, especially likely when the bodily vessel diameter is less than 4mm. For example, when a covered stent is introduced to treat an intracranial aneurysm, occlusion of the parent artery is still likely to occur even though the aneurysm is covered, due to the small vessel diameter of the artery.

### Summary of the Invention

In a first preferred aspect, there is provided an endovascular device for treatment of a bodily vessel of a patient at a surgical site, the endovascular device comprising:
a mechanically expandable device expandable from a first position to a second position, said mechanically expandable device is expanded radially outwardly to the second position such that the circumferential surface of said mechanically expandable device engages with the inner surface of the vessel so as to maintain a fluid pathway through said vessel; and
a membrane secured to a portion of the circumferential surface of said mechanically expandable device;
wherein at least one capture agent is permanently attached to the lumenal surface of the membrane to capture a predetermined target component from blood passing through the fluid pathway; and at least one signal agent is permanently attached to the lumenal surface of the membrane to signal the captured target component to up regulate or down regulate a cell function of the captured target component to enhance endothelialization and healing.

The cell function may be any one from the group consisting of: proliferation, migration, maturation, and apoptosis.

The predetermined target component may be an endothelial progenitor cell, and the signal agent up regulates the proliferation and maturation of the endothelial progenitor cell to increase the rate of endothelialization and reduce the time for inflammation and thrombosis.

The signal agent may be an endothelial progenitor cell specific L-PDMP.

The signal agent may be arranged in a first conformation of a single arm structure to function independently of the capture agent.

The signal agent down may regulate the cell function of a cell to reduce proliferation or up regulate the apoptosis of undesirable cells adsorbed on the surface of the membrane.

The signal agent may be SMC-specific D-PDMP.

There may be included at least one signal agent permanently attached to the vessel wall surface of the membrane to signal a predetermined target component from the vessel wall in contact with the membrane to up regulate or down regulate a cell function to enhance healing of the vessel wall and incorporation of the device into the vessel wall.

The cells may be any one from the group consisting of: fibroblast cells and smooth muscle cells.

The cell function may be any one from the group consisting of: production of protein chains and proliferation.

There may be included at least one capture agent permanently attached to the vessel wall surface of the membrane to capture the predetermined target component from the vessel wall in contact with the membrane.

The cell function may be any one from the group consisting of: proliferation, migration, maturation and apoptosis.

The signal agent may be an anti-inflammatory agent and the cell function is to reduce the recruitment and infiltration of white blood cells.

The signal agent may enhance endothelial cell alignment and proliferation of endothelial cells to the membrane.

The capture agent and signal agent may increase the rate of endothelialization to decrease the time for the reaction of thrombosis and restenosis to occur after the mechanically expandable device is expanded to the second position.

The capture agent may be arranged in a first conformation of a single arm structure made up of an organic linker anchored to the membrane.

The capture and signal agents may be arranged in a second conformation of a branched structure made up of an organic linker anchored to the membrane.

The membrane may be made from either a biostable or a biodegradable material. The membrane may be solid or permeable.

The capture and signal agents may be any one from the group consisting of: enzyme regulators tagged with antibodies or peptides, L-PDMP, peptides, antibodies, naturally occurring molecules, and synthetic molecules.

The membrane may include an intermediate layer to mimic the basal lamina such that endothelial cells form a strong bond with the membrane.

The membrane may be secured to the entire circumferential surface of the mechanically expandable device.

The bodily vessel may be any one from the group consisting of: intracranial bodily vessel, bodily vessel suffering from a hemorrhagic, ischemic or vascular disease, bodily vessel with damaged vessel walls, and bodily vessel less than 4 mm in diameter.

In a second aspect, there is provided an endovascular device for treatment of a bodily vessel of a patient at a surgical site, the endovascular device comprising:
a mechanically expandable device expandable from a first position to a second position, said mechanically expandable device is expanded radially outwardly to the second position such that the circumferential surface of said mechanically expandable device engages with the inner surface of the vessel so as to maintain a fluid pathway through said vessel; and
a membrane secured to a portion of the circumferential surface of said mechanically expandable device;
wherein at least one capture agent is permanently attached to the vessel wall surface of the membrane to enhance healing of the vessel wall from injury caused after the mechanically expandable device is expanded to the second position and to enhance healing of the vessel wall and incorporation of the device into the vessel wall.

The capture agent may enable proliferation of vessel wall components to enhance the healing of the weakened or breached portion of the vessel wall.

The capture agent may encourage proliferation and bridging of vessel wall components across a weakened, torn or breached portion of the vessel wall to enhance healing and encasement of the device at a portion of the device in contact with the vessel wall.

The capture agent may be arranged in a first conformation of a single arm structure made up of an organic linker anchored to the membrane.

The capture agent may be arranged in a second conformation of a branched structure made up of an organic linker anchored to the membrane.

In a third aspect, there is provided an endovascular device for treatment of a bodily vessel of a patient at a surgical site, the endovascular device comprising:
a mechanically expandable device expandable from a first position to a second position, said mechanically expandable device is expanded radially outwardly to the second position such that the circumferential surface of said mechanically expandable device engages with the inner surface of the vessel so as to maintain a fluid pathway through said vessel; and
wherein at least one capture agent is permanently attached to the lumenal side of the device to capture a predetermined target component from blood passing through the fluid pathway, and at least one signal agent is permanently attached to the lumenal side of the device to signal the captured target component to up regulate or down regulate a cell function of the captured target component to enhance endothelialization and healing; and
a third agent is permanently attached to the vessel wall side of the device to enhance healing of the vessel wall from injury caused after the mechanically expandable device is expanded to the second position.

A membrane may be secured to a portion of the circumferential surface or entire circumferential surface of said mechanically expandable device; the first, second and third agents being permanently attached to the membrane.

The third agent may be arranged in a first conformation of a single arm structure made up of an organic linker anchored to the membrane.

The third agent may be arranged in a second conformation of a branched structure made up of an organic linker anchored to the membrane.

The third agent may enable proliferation of vessel wall components to enhance the healing of the weakened or breached portion of the vessel wall.

### Brief Description of the Drawings

An example of the invention will now be described with reference to the accompanying drawings, in which:

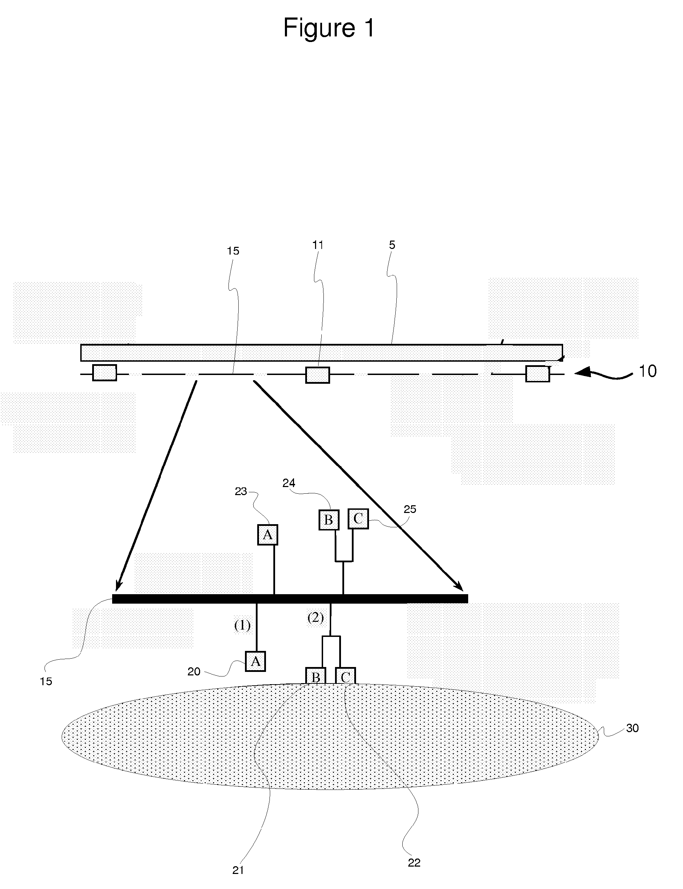

Figure 1 is an illustration of a preferred embodiment of the present invention deployed in a bodily vessel.

### Detailed Description of the Drawings

Referring to Figure 1, there is provided an endovascular device 10 for treatment of an intracranial bodily vessel 5 of a patient at a surgical site. The bodily vessel 5 may suffer from hemorrhagic, ischemic or vascular diseases or have weakened, torn or breached vessel walls. The device 10 comprises: a stent-like scaffold 11 covered by the ultra-thin membrane or coating 15. The membrane 15 may be a solid or permeable membrane 15 with varying degrees of porosity. The membrane 15 has two surfaces: a lumenal surface and a vessel wall surface. On the lumenal surface, agents 20, 21, 22 are permanently attached to the membrane 15. On the vessel wall surface, agents 23, 24, 25 are permanently attached to the membrane 15.

At least one capture agent 21 is permanently attached to the lumenal surface of the membrane 15 to capture a predetermined target component 30 from blood passing through the fluid pathway. At least one signal agent 22 is permanently attached to the lumenal surface of the membrane 15 to signal the captured target component 30 to up regulate or down regulate a cell function of the captured target component 30 to enhance endothelialization and healing. The cell function may include: proliferation, migration, maturation, and apoptosis. The predetermined target component 30 may include an endothelial progenitor cell 30, and the signal agent 22 up regulates the proliferation and maturation of the endothelial progenitor cell 30 to increase the rate of endothelialization and reduce the time for inflammation and thrombosis. The capture and signal agents 21, 22 include: enzyme regulators tagged with antibodies or peptides, L-PDMP, peptides, antibodies, naturally occurring molecules, and synthetic molecules. Specifically, the signal agent 22 may be an endothelial progenitor cell specific L-PDMP or an SMC-specific D-PDMP. The signal agent 22 may also be an anti-inflammatory agent and the cell function is to reduce the recruitment and infiltration of white blood cells. This signal agent 22 enhances endothelial cell alignment and proliferation of endothelial cells 30 to the membrane 15. 15. The capture agent 21 and signal agent 22 increase the rate of endothelialization to decrease the time for the reaction of thrombosis and restenosis to occur after the stent 11 is expanded.

The capture agent 21 and signal agent 22 are arranged in a conformation of a branched structure made up of an organic linker anchored to the membrane 15. Alternatively, the signal agent 22 may be arranged in a conformation of a single arm structure to function independently of the capture agent 21. This single arm structure is made up of an organic linker anchored to the membrane 15.

The signal agent 22 may also down regulate the cell function of a cell to reduce proliferation or up regulate the apoptosis of undesirable cells adsorbed on the surface of the membrane. In this case, the cell is not only limited to the captured target component 30.

At least one signal agent 25 is permanently attached to the vessel wall surface of the membrane 15. The signal agent 25 signals a predetermined target component from the vessel wall 5 in contact with the membrane 15 to up regulate or down regulate a cell function to enhance healing of the vessel wall 5 and incorporation of the device 10 into the vessel wall 5. The cells include: fibroblast cells and smooth muscle cells. The cell function includes: production of protein chains and proliferation.

At least one capture agent 24 is permanently attached to the vessel wall surface of the membrane 15. The capture agent 24 captures the predetermined target component from the vessel wall 5 in contact with the membrane 15. The cell function includes: proliferation, migration, maturation and apoptosis.

Typically, when an implant such as a stent 11 is placed into a vessel 5 to exact an appropriate therapeutic effect, for example, placed into an intracranial vessel 5 to bridge the neck of an aneurysm or cover a hemorrhage, it breaks up surrounding healthy endothelium and injures the vessel wall 5. The injury induces immune responses like thrombosis and inflammation, which leads to smooth muscle cell proliferation and sometimes, occlusion of the vessel 5. The occlusion problem is exacerbated in small vessels 5, such as those found in the intracranial region.

### Membrane

The membrane 15 is secured to a portion of the circumferential surface of the stent 11. In another embodiment, the membrane 15 may be secured to the entire circumferential surface of the stent 11. The membrane 15 is made from materials, for example, polymeric materials, with tailored-made and pre-designed surface characteristics. A biostable material or a biodegradable material is suitable for the membrane 15. For biostable non-erodable materials, examples are poly-urethanes and poly-urethane based polymers like poly-urethane ethers and poly-urethane esters, polymethyl-methacrylate (PMMA), silicone, ethyl vinyl alcohol, polystyrene, polyolefin, polyester, polyamide, fluorocarbons such as ePTFE and PTFE as well as mixtures and copolymers of the above mentioned. For biodegradable polymers, examples are Poly (glycolic acid) (PGA), Poly (lactic acid) (PLA), Poly (lactic-co-glycolic acid) (PLGA), poly (ecaprolactone), Polyanhydride, poly (orthoesters), polyphosphazane; biodegradable polymers from natural sources such as modified polysaccharides (cellulose, chitin, dextran) and Modified proteins (fibrin, casein); and hydrogels or superabsorbants such as Poly (ethylene oxide) (PEO), Poly (ethylene glycol) PEG, Methylacrylate (MAA), Maleic anhydride (MAH), Polyacrylamide, Poly (hydroxyethyl methacrylate), Poly (N-vinyl pyrrolidone), Poly (vinyl alcohol).

The material surface of the membrane 15 is modified to minimize non-specific interactions with and adhesion of components in blood that are involved in undesirable inflammation or thrombosis. For example, these components include white blood cells, platelets, fibrin, cytokines, growth factors, enzymes, other proteins and peptides, and smooth muscle cells. The surface of the membrane 15 is able to prevent non-specific adhesion of unwanted components in blood depends on a range of factors. Factors may include the type of grafted functional groups or end-groups, for example, fluorocarbons and silicone end groups; concentration and distribution of the grafted groups; overall hydrophobicity of the surface of the membrane 15 associated with surface free energy, crystallinity and ratio of hard and soft segments; surface homogeneity; and ionic charge of the surface. The surface of the membrane 15 at the lumen side is capable of selective interaction with components found in blood. There are mechanisms provided for selective communication with target components found in blood. Also, the surface of the membrane 15 at the vessel wall side is capable of selective interaction with vessel wall components.

The surface characteristics of the membrane 15 maintain patency of the vessel 5 in which the device 10 is implanted, by reducing, minimizing, preventing or completely avoiding the negative immune reactions against placement of a foreign body 10 in the vessel 5 while encouraging the healing of the endothelium and incorporation of the device 10 into the vessel wall 5.

Also, the modifications of the surface of the membrane 15 allow the pharmaceutical agents 20, 21, 22, 23, 24, 25 to be attached via linker molecules. These attached agents target and bind strongly to desirable components required for enhanced endothelialization (for example, endothelial progenitor cells 30 in the blood stream), vessel healing and incorporation of device 10 into vessel wall 5. The attached agents can also function to signal or communicate with the bound components to further enhance endothelialization and healing responses.

One example of a material for the membrane 15 is a PU-based material with surface-modifying end-groups, either fluorocarbons or PEO or both. Fluorocarbon and PEO SMEs prevent adhesion of undesirable blood components involved in inflammation and thrombosis. By including a variety of PEO SMEs (with different length, polarity, etc), pharmaceutical agents can be attached to the surface to capture EPC for enhanced endothelialization and healing.

The surfaces of the membrane or coating 15 have a dual purpose. Firstly, they minimize interaction with and adhesion of undesirable components in blood associated with inflammation and thrombosis. Secondly, they comprise permanently bound agents 20, 21, 22, 23, 24, 25 that specifically target and bind to certain desirable components in blood associated with endothelialization and healing. The agents include agents that able to signal or communicate with the captured components to further enhance endothelialization or healing. This may bring about faster endothelialization and healing, for example, if L-PDMP is used.

As a result of the surface characteristics of the membrane or coating 15, the following are enhanced: healing, endothelialization and incorporation of the device 10 into the vessel wall 5. Adverse immune responses reacting to implantation of the foreign body is significantly reduced. Hence, the patency of the vessel 5 in which the device 10 is implanted is maintained.

### Agents

The pharmaceutical agents 20, 21, 22 coated on the lumen side of the membrane 15 prevent the occlusion of the original patent lumen. A pharmaceutical capture agent 21 is permanently attached to the lumenal surface of the membrane 15 to capture progenitor endothelial cells 30 from blood. A pharmaceutical signal agent 22 is permanently attached to the lumenal surface of the membrane 15 to enhance endothelial cell alignment and proliferation to the membrane 15. The capture and signal agents 20, 21, 22 increase the rate of endothelialization to decrease the time for the reaction of thrombosis and restenosis to occur after the stent 11 is deployed.

In a preferred embodiment, the capture agent 21 is arranged in a first conformation of a single arm structure made up of an organic linker anchored to the membrane 15. The organic linker may be a short chain of organic molecules anchored on one end to the membrane 15 and the other end bound to the agent molecule that captures specifically endothelial progenitor cells 30 from the blood to promote endothelialization. The capture and signal agents 20, 21, 22 are arranged in a second conformation of a branched structure made up of an organic linker anchored to the membrane 15. The capture agent 21 specifically captures endothelial progenitor cells similar to the other capture agent 20 while a signal agent 22 enhances endothelial cell alignment and proliferation.

Alternatively, the signal agent 22 is arranged in a first conformation of a single arm structure made up of an organic linker anchored to the membrane 15.

On the vessel wall side of the membrane 15, a third pharmaceutical agent 23 is permanently attached to the vessel wall surface of the membrane 15 to enhance healing of the vessel wall 5 from injury caused after the stent 11 is deployed. Alternatively, the agents on the vessel wall side of the membrane 15 also encourage proliferation of vessel wall components, for example, intima, intima elastic lamina (IEL), for enhancing the healing of the weakened or breached portion of the vessel wall, for example, an aneurysm neck. The agents also bring about encasement of the device 10 from the vessel wall side. This is in contrast to encasement of the device 10 from the lumen side which is via endothelialization. The third agent 23 is arranged in a first conformation of a single arm structure made up of an organic linker anchored to the membrane 15. Alternatively, the capture agent 24 is arranged in a second conformation of a branched structure made up of an organic linker anchored to the membrane 15. The second conformation may also have a signal agent 25 on the other branch.

The device 10 is used for treatment of intracranial aneurysms, small vessels with hemorrhage for example, small intracranial vessels with hemorrhage, and other endovascular diseases. The device 10 comprises a stent-like scaffold 11. A portion of the scaffold is covered by ultra-thin membrane or coating 15. The surfaces of the membrane or coating 15 are coated with pharmaceutical agents 20, 21, 22 to reduce, minimize, prevent or completely avoid the negative immune reactions against foreign body placement in the vessel and encourage healing of the endothelium quickly.

In another embodiment, the membrane 15 is optional for the device 10. A capture agent 21 is permanently attached to the lumenal side of the device 10 to capture progenitor endothelial cells 30 from blood passing through the fluid pathway. A signal agent 22 is permanently attached to the lumenal side of the device 10 to enhance endothelial cell alignment and proliferation. The capture and signal agents 21, 22 increase the rate of endothelialization to decrease the time for the reaction of thrombosis and restenosis to occur after the stent 11 is expanded to the second position. A third agent 23 is permanently attached to the vessel wall side of the device 10 to enhance healing of the vessel wall from injury caused after the stent 11 is expanded to the second position.

For example, for intracranial aneurysms, the placement of the device 10 treats the aneurysm while the pharmaceutical agents on the surface of the device 10 minimize immune response due to device implantation and vessel injury. The agents also optimize healing and endothelialization.

The therapeutic effects of the agents 20, 21, 22 on the lumen side of the membrane 15 enhance endothelialization. For example, the agents 20, 21, 22 specifically attract and capture endothelial cells 30 and/or endothelial progenitor cells 30 from the blood stream to aid in formation of a healthy endothelium. Multiple agents 20, 21, 22 are coated on the lumen side. For example, a capture agent 21 to capture the endothelial and endothelial progenitor cells 30 and a signal agent 22 to enhance proliferation of the endothelial cell 30 after binding to the capture agent 21. The lumen side of the membrane 15 generally discourages white blood cells, platelets, fibrin, cytokines, growth factors, smooth muscle cells, enzymes, other cell types, as well as other molecules and compounds involved in thrombosis and inflammation in the blood stream from binding to the lumen side of the membrane 15.

The types of agents 20, 21, 22 for the lumen side surface of the membrane 15 include: enzymes regulators, peptides or antibodies, naturally occurring molecules and synthetic molecules. Enzymes regulators are tagged with antibodies, peptides or other compounds. Enzyme regulators such as L-threo-PDMP (L-threo-1-Phenyl-2-decanoylamino-3-morpholino-1-propanol) can be conjugated with tags (for example, antibodies or peptides) that can bind uniquely and specifically to target molecules on the surface of endothelial and endothelial progenitor cells 30. When an endothelial or endothelial progenitor cell is captured it undergoes proliferation enhanced by the adjacent enzyme regulator, to increase the rate of endothelialization. Peptides or antibodies have high binding affinity and specificity for endothelial cells or endothelial progenitor cells 30. Naturally occurring molecules (synthesized or purified) can mimic part of the basal lamina of the endothelium, so that the endothelial or endothelial progenitor cells 30 in the bloodstream will preferentially and uniquely bind and anchor to the lumen surface of the membrane 15, and form good cell alignment on the lumen surface of the membrane 15 and proliferate to confluence. For example, laminin-mimetic pentapeptide immobilized on the lumen surface capture and binds to endothelial or endothelial progenitor cells 30 in the blood stream. The naturally occurring molecules have very low binding affinity for other molecules and cells associated with thrombosis and inflammation. Novel synthetic molecules enhance endothelialization, whereas other synthesized molecules are designed to mimic naturally occurring molecules with the function of enhancing endothelialization.

An intermediate layer may be provided on lumen side of the membrane 15 that mimics the basal lamina above the internal elastic lamina. The basal lamina is a thin sheet-like network of ECM components made up of laminins and collagen on which the endothelial cells 30 are situated. It is envisaged that endothelial or endothelial progenitor cells 30 in the blood stream bind strongly to the lumen side of the membrane 15 and proliferate as though they were on a natural basal lamina.

The therapeutic effects of the agents 23, 24, 25 on the vessel wall side of the membrane 15 enhance vessel wall recovery from injury due to implantation of the device 10 and device adherence and incorporation to the vessel wall surface. The target molecule is a molecule to which the therapeutic agents have a high binding affinity. For example, a receptor on the surface of endothelial progenitor cell 30 is referred to as the target molecule/component. Alternatively, the agent may encourage proliferation and bridging of vessel wall components across the weaken, torn or breached portion of the vessel wall 5, for example, the neck of an aneurysm on the vessel wall side, for enhanced healing and encasement of the device 10 from the wall side.

The types of agents for the vessel wall side of the membrane 15 include: synthesized molecules (based on naturally occurring compounds) or novel synthetic molecules designed to enhance the healing of the injured wall and incorporation of the device 10 within the vessel wall 5. To encourage encasement of device 10 by the vessel 5, enzyme regulators like L-threo-PDMP (L-threo-1-Phenyl-2-decanoylamino-3-morpholino-1-propanol) may be used to up-regulate the proliferation of smooth muscle cells and expedite the incorporation of the device 10 into the part of the vessel where the wall 5 is weakened, torn or breached.

The agents are bound to the linker. The linker is permanently bound to the surface of the membrane 15 such that the orientation and availability are optimized and the chemical structure and properties (such as binding affinity and specificity) are retained, in order to perform the desired therapeutic functions. Therapeutic functions include to efficient capture of endothelial cells and endothelial progenitor cells 30 from blood passing by the lumen side, and cells in the vessel wall 5 on the vessel wall side.

The concentration of the agents on either the lumen side or vessel wall side of the device 10 is optimized to levels in order to perform their respective therapeutic functions effectively. Distribution of the agents on the surface of the membrane 15 is selected to optimized therapeutic effect.

The molecular structure and size of the agents and their corresponding linkers are modified to optimize binding availability, binding affinity and specificity to their targets, for example, molecules on the endothelial cell surface 30, endothelial progenitor cell surface 30 or other molecules involved in healing.

The bonds between agent and linker as well as linker and surface of the membrane 15 are stable and not prone to hydrolysis. There are minimal unintended interactions among the agents, linkers and surface of the membrane 15 that may result in compromising the availability, binding affinity, binding specificity and other chemical properties of the agents.

Thickness of the membrane or coating 15 over the stent-like scaffold 11 inclusive of the surface-bound pharmaceutical agents (single side or both sides) should be between 5 - 100 µm max.

In an example where the bodily vessel 5 is not technically diseased but has a portion of the vessel wall which is weakened, torn or breached, the device 10 may be used to cover or bridge the torn or breached portions. This leads to a new vessel wall and endothelium to be regenerated at these weakened portions.

In one embodiment, the device 10 only has at least one capture agent 24 permanently attached to the vessel wall surface of the membrane 15 to enhance healing of the vessel wall 5 from injury caused after the stent 11 is expanded. The capture agent 24 enhances healing of the vessel wall and incorporation of the device 10 into the vessel wall 5. The capture agent 24 also enables proliferation of vessel wall components to enhance the healing of the weakened or breached portion of the vessel wall 5. The capture agent 24 also encourages proliferation and bridging of vessel wall components across a weakened, torn or breached portion of the vessel wall 5 to enhance healing and encasement of the device 10 at a portion of the device 10 in contact with the vessel wall 5.

In another embodiment, the device 10 may not have a membrane 15 for attachment of the agents. The capture agent 21 is permanently attached to the lumenal side of the device 10. The signal agent 22 is also permanently attached to the lumenal side of the device 10. A third agent 25 is permanently attached to the vessel wall side of the device 10 to enhance healing of the vessel wall from injury caused after the stent 11 is expanded. The third agent 25 also enables proliferation of vessel wall components to enhance the healing of the weakened or breached portion of the vessel wall 5. If a membrane 15 is used by the device 10, the third agent 25 is arranged in a first conformation of a single arm structure made up of an organic linker anchored to the membrane 15. Alternatively, the third agent 25 is arranged in a second conformation of a branched structure made up of an organic linker anchored to the membrane 15.

Although a device 10 has been described for use in small vessels, the invention is envisaged to cover intracranial graft for intracranial aneurysms, and stent grafts.

It will be appreciated by persons skilled in the art that numerous variations and/or modifications may be made to the invention as shown in the specific embodiments without departing from the scope or spirit of the invention as broadly described. The present embodiments are, therefore, to be considered in all respects illustrative and not restrictive.

## Claims

1. An endovascular device for treatment of a bodily vessel of a patient at a surgical site, the endovascular device comprising:
a mechanically expandable device expandable from a first position to a second position, said mechanically expandable device is expanded radially outwardly to the second position such that the circumferential surface of said mechanically expandable device engages with the inner surface of the vessel so as to maintain a fluid pathway through said vessel; and
a membrane secured to a portion of the circumferential surface of said mechanically expandable device;
wherein at least one capture agent is permanently attached to the lumenal surface of the membrane to capture a predetermined target component from blood passing through the fluid pathway; and at least one signal agent is permanently attached to the lumenal surface of the membrane to signal the captured target component to up regulate or down regulate a cell function of the captured target component to enhance endothelialization and healing.

2. The device according to claim 1, wherein the cell function is any one from the group consisting of: proliferation, migration, maturation, and apoptosis.

3. The device according to claim 1, wherein the predetermined target component is an endothelial progenitor cell, and the signal agent up regulates the proliferation and maturation of the endothelial progenitor cell to increase the rate of endothelialization and reduce the time for inflammation and thrombosis.

4. The device according to claim 3, wherein the signal agent is an endothelial progenitor cell specific L-PDMP.

5. The device according to claim 1, wherein the signal agent is arranged in a first conformation of a single arm structure to function independently of the capture agent.

6. The device according to claim 5, wherein the signal agent down regulates the cell function of a cell to reduce proliferation or up regulate the apoptosis of undesirable cells adsorbed on the surface of the membrane.

7. The device according to claim 6, wherein the signal agent is SMC-specific D-PDMP.

8. The device according to claim 1, further comprising at least one signal agent permanently attached to the vessel wall surface of the membrane to signal a predetermined target component from the vessel wall in contact with the membrane to up regulate or down regulate a cell function to enhance healing of the vessel wall and incorporation of the device into the vessel wall.

9. The device according to claim 8, wherein the cells are any one from the group consisting of: fibroblast cells and smooth muscle cells.

10. The device according to claim 8, wherein the cell function is any one from the group consisting of: production of protein chains and proliferation.

11. The device according to claim 8, further comprising at least one capture agent permanently attached to the vessel wall surface of the membrane to capture the predetermined target component from the vessel wall in contact with the membrane.

12. The device according to claim 11, wherein the cell function is any one from the group consisting of: proliferation, migration, maturation and apoptosis.

13. The device according to claim 1, wherein the signal agent is an anti-inflammatory agent and the cell function is to reduce the recruitment and infiltration of white blood cells.

14. The device according to claim 1, wherein the signal agent enhances endothelial cell alignment and proliferation of endothelial cells to the membrane.

15. The device according to claim 14, wherein the capture agent and signal agent increase the rate of endothelialization to decrease the time for the reaction of thrombosis and restenosis to occur after the mechanically expandable device is expanded to the second position.

16. The device according to claim 1, wherein the capture agent is arranged in a first conformation of a single arm structure made up of an organic linker anchored to the membrane.

17. The device according to claim 1, wherein the capture and signal agents are arranged in a second conformation of a branched structure made up of an organic linker anchored to the membrane.

18. The device according to claim 1, wherein the membrane is made from either a biostable or a biodegradable material.

19. The device according to claim 1, wherein the membrane is solid or permeable.

20. The device according to claim 1, wherein the capture and signal agents are any one from the group consisting of: enzyme regulators tagged with antibodies or peptides, L-PDMP, peptides, antibodies, naturally occurring molecules, and synthetic molecules.

21. The device according to claim 1, wherein the membrane includes an intermediate layer to mimic the basal lamina such that endothelial cells form a strong bond with the membrane.

22. The device according to claim 1, wherein the membrane is secured to the entire circumferential surface of the mechanically expandable device.

23. The device according to claim 1, wherein the bodily vessel is any one from the group consisting of: intracranial bodily vessel, bodily vessel suffering from a hemorrhagic, ischemic or vascular disease, bodily vessel with damaged vessel walls, and bodily vessel less than 4 mm in diameter.

24. An endovascular device for treatment of a bodily vessel of a patient at a surgical site, the endovascular device comprising:
a mechanically expandable device expandable from a first position to a second position, said mechanically expandable device is expanded radially outwardly to the second position such that the circumferential surface of said mechanically expandable device engages with the inner surface of the vessel so as to maintain a fluid pathway through said vessel; and
a membrane secured to a portion of the circumferential surface of said mechanically expandable device;
wherein at least one capture agent is permanently attached to the vessel wall surface of the membrane to enhance healing of the vessel wall from injury caused after the mechanically expandable device is expanded to the second position and to enhance healing of the vessel wall and incorporation of the device into the vessel wall.

25. The device according to claim 24, wherein the capture agent enables proliferation of vessel wall components to enhance the healing of the weakened or breached portion of the vessel wall.

26. The device according to claim 24, wherein the capture agent encourages proliferation and bridging of vessel wall components across a weakened, torn or breached portion of the vessel wall to enhance healing and encasement of the device at a portion of the device in contact with the vessel wall.

27. The device according to claim 24, wherein the capture agent is arranged in a first conformation of a single arm structure made up of an organic linker anchored to the membrane.

28. The device according to claim 24, wherein the capture agent is arranged in a second conformation of a branched structure made up of an organic linker anchored to the membrane.

29. An endovascular device for treatment of a bodily vessel of a patient at a surgical site, the endovascular device comprising:
a mechanically expandable device expandable from a first position to a second position, said mechanically expandable device is expanded radially outwardly to the second position such that the circumferential surface of said mechanically expandable device engages with the inner surface of the vessel so as to maintain a fluid pathway through said vessel; and
wherein at least one capture agent is permanently attached to the lumenal side of the device to capture a predetermined target component from blood passing through the fluid pathway, and at least one signal agent is permanently attached to the lumenal side of the device to signal the captured target component to up regulate or down regulate a cell function of the captured target component to enhance endothelialization and healing; and
a third agent is permanently attached to the vessel wall side of the device to enhance healing of the vessel wall from injury caused after the mechanically expandable device is expanded to the second position.

30. The device according to claim 29, further comprising a membrane secured to a portion of the circumferential surface or entire circumferential surface of said mechanically expandable device; the first, second and third agents being permanently attached to the membrane.

31. The device according to claim 29, wherein the third agent is arranged in a first conformation of a single arm structure made up of an organic linker anchored to the membrane.

32. The device according to claim 29, wherein the third agent is arranged in a second conformation of a branched structure made up of an organic linker anchored to the membrane.

33. The device according to claim 29, wherein the third agent enables proliferation of vessel wall components to enhance the healing of the weakened or breached portion of the vessel wall.
